# EUROPEAN PATENT SPECIFICATION

(11) **EP 2 383 008 B1**
(45) Date of publication and mention of the grant of the patent: **06.11.2013**
(21) Application number: 10161547.4
(22) Date of filing: 30.04.2010
(51) Int. Cl.: A61M 16/08, A61M 16/00

(54) **Arrangement for maintaining volume of breathing gas in a desired level**
Anordnung zur Aufrechterhaltung der Menge von Atemgas auf einem gewünschten Niveau
Agencement pour maintenir le volume d'un gaz respiratoire à un niveau souhaité

(43) Date of publication of application: 02.11.2011
(73) Proprietor: General Electric Company, Schenectady, NY 12345 (US)
(72) Inventor: Haveri, Heikki Antti Mikael, 03150 Huhmar (FI)
(74) Representative: Kanerva, Arto

(56) References cited:
- EP-A1- 2 168 623
- WO-A1-2009/062540
- US-A- 5 050 615
- US-A- 6 142 150
- US-A1- 2008 091 117

## Description

### BACKGROUND OF THE INVENTION

This disclosure relates generally to a method and an arrangement for maintaining a volume of a breathing gas in a desired level when ventilating a subject.

A tidal volume (TV) is an amount of an air inspired or taken into lungs in a single breath. TV is dependent on the sex, size, height, age and a health etc. of a patient, but in general TV also decreases as the size of the patient decreases. In an average healthy adult, TV is about 400-600 ml whereas in an average healthy neonate, that measures 3.5-4 kg and is 50 cm tall, TV is approximately 25-50 ml. On the other hand, in an average premature neonate that measures only 500 grams TV is only about 2-3.5 ml. TV of a smaller patient's is very difficult to measure, but it can be approximated to 4-7 ml/kg, applying a general rule of thumb for approximating the TV of the human lung. In practice the TV of the patient suffering pulmonary system deficiency is normally much less than the approximation gives.

A respiration rate (RR) is also dependent on the sex, size, height, age and a health etc. of the patient, but in general RR increases as the size of the patient decreases. In an average healthy adult, RR is about 10-20 breaths/minute, whereas RR of a neonate may exceed as high as 150 breaths/minute.

When the patient is mechanically ventilated with a conventional ventilator, an endotracheal tube is placed into a trachea so that it goes through oral or nasal cavity and larynx. The other end of the endotracheal tube is connected to a breathing circuit Y-piece through a luer type connector. If the patient is gas monitored with a mainstream or sidestream gas analyzer, an airway adapter used for sampling the breathing gas that is analyzed by the gas analyzer is normally connected between connectors of the endotracheal tube and the breathing circuit Y-piece. During an inspiration the fresh breathing gas including higher oxygen (O₂) concentration flows into the patients lungs through an inspiratory limb of the breathing circuit Y-piece, the airway adapter, the endotracheal tube and their connectors, then to a trachea, a bronchus, a bronchi, bronchioles and finally reaching an alveoli deep in the lungs, where all the gas exchange actually occurs. Carbon dioxide (CO₂) molecules in a hemoglobin of a blood flowing in tiny blood vessels around the alveoli are replaced with O₂ molecules in the fresh breathing gas through the thin walls of the alveoli. O₂ molecules take their place in the hemoglobin, whereas CO₂ molecules flow out from the patient within the used expired breathing gas, through the same path as the fresh gas came in during the inspiration. Thus a gas concentration of the breathing gas measured by the gas analyzer is somewhat proportional to the gas concentration in the blood.

A volume in a space between a connection of the inspiratory and expiratory limbs of the Y-piece and the patient's mouth or nose, a beginning of oral and nasal cavities, is called a mechanical dead volume or dead space, whereas the volume in a space between patient's mouth or nose and the entrance of alveoli is called an anatomical dead volume. The part of the lung that is injured or damaged for some reason and does not participate for the gas exchange is called more specific a physical dead volume. It is obvious that as the used breathing gas flows out from the patient's lungs through the expiratory limb during expiration, a part of the used gas newer exits a pulmonary system, as well as the patient side of the breathing circuit, but remains in the mechanical and anatomical dead volume. Then as the fresh gas is inspired in to the lungs through the inspiratory limb the used gas already in the anatomical and mechanical dead volume flows into the lungs before the fresh gas. The used gas fills up some or all of the alveoli depending on a ratio of the dead volume and TV or at least mixes up with the fresh gas decreasing the concentration of O₂ as well as increasing the concentration of CO₂ in the lungs, which in turn decreases the gas exchange in the alveoli. This means that the larger the dead space, the larger the volume of the used gas, with a low O₂ and high CO₂ concentration, that flows back to the patients lungs during the inspiration and worse the gas exchange in the alveoli. In other words, if the total dead volume were larger than TV or as large as TV, the patient would not get any fresh gas into the lungs, but respires the used gas back and forth in the dead volume. In practice a diffusion of gases assists the gas exchange over the dead volume little, especially when there is some movement of gases such as a high frequency ventilation evolved, but the overall gas exchange in the alveoli would be lethal or dangerously poor anyway.

The anatomical dead volume is almost impossible to reduce, but it is proportional to the size and the physical condition of the patient. The mechanical dead volume depends on a breathing circuit design, an inner diameter of breathing circuit tubing, connectors and additional accessories, such as airway adapters used with a sidestream and mainstream gas analyzers. Obviously it is optimal that the mechanical dead space is zero as with normal breathing. It is also obvious that the mechanical dead volume is more critical for smaller patients with smaller TV or patients suffering barotraumas etc., which decrease TV.

The mainstream gas analyzing is suitable for intubated patients or patients wearing face mask or nasal mask in general. Mainstream analyzers are placed between the breathing circuit Y-piece and endotracheal tube through their airway accessory used for measuring the gas concentration of the gas flowing through the analyzer. However, existing mainstream gas analyzers are big and heavy and thus very impractical to use especially with small patients as they cover the patients face and tiny endotracheal tube easily bents and clock under the weight. Furthermore accessories and additional connectors add the dead space considerably, which is critical for a small patient with small TV. Also the design of airway adapter and its non-tubular gas sampling chamber is inefficient and generates turbulences in to the breathing gas flow mixing end tidal gas with fresh gas, thus mixing the gas samples that the mainstream analyzer measures, causing measurement inaccuracy especially with higher RR and small TV. In practice existing mainstream analyzers are not used with smaller patients at the moment at all.

The sidestream gas analyzing can be used with intubated and non-intubated patients. Gas samples are sucked actively into the analyzer with a gas pump through a sampling tubing. When measuring intubated patients, the sampling tubing is connected to airway adapter placed between the breathing circuit Y-piece and endotracheal tube to take samples from the breathing gas flowing inside the breathing circuit. When measuring non-intubated patients the sampling tubing can be connected to for example a nasal cavity to take gas samples straight from patient's upper airways.

The airway adapter placed close to the patient is usually small and light and thus practical to use, whereas the big and heavy analyzer itself is located further away from a patient, for example inside the patient monitor or ventilator. However, the distance between the patient and the analyzer means that gas samples travel a long way before entering the analyzer starting from the airway adapter, through several mechanical connections between different parts of the sampling circuit, tiny tubing, which is usually 3-6 m long with inner diameter between 1-1.5 mm, and finally through filters that separate water, mucus, blood etc. As gas samples, or columns of the gas including different gas concentrations, travel through connectors, sample tubing and filters, they mix up and average along the long path degrading the gas concentration measurement accuracy considerably. Furthernore, the measurement accuracy degrades rapidly, especially when RR increases and TV degreases. This can be seen as damped and rounded capnogram, which is due to smaller samples or shortened gas columns that mix up and average easier causing the amplitude to decrease rapidly. Also the flow rate of the sample gas has an effect on gas sample averaging. The lower the sample gas flow, the longer the gas columns travel through the tubing etc. and the more they mix up and average. Sample gas flow rates of existing sidestream gas analyzers are usually between 50-400 ml/min. According to the laws of physics it is obvious that as the flow rate of the sample gas is decreased for example from 200 ml/min to 50 ml/min the sensitivity to breathing gas concentration changes decreases not proportionally, but exponentially as the sample gas travels longer inside the tubing etc. and mixes up and averages even more. For that reason most of the sidestream gas analyzer manufacturers only specify the measurement range for RR, which may go up frequencies of 120-150 breaths/minute, but the accuracy of the gas concentration measurement is not specified or it is specified only up to 15-60 breaths/minute, which is usable only for adults.

In many cases the flow rate of sample gas cannot be increased to high enough levels to get sufficient sensitivity for the gas concentration measurement. The sample gas is "stolen" from the fresh inspratory gas that should flow in to the patient and it decreases the volume of the inspiratory air, which in turn decreasing the gas exchange in the alveoli. Thus existing gas analyzers can usually measure the concentration of the gas for RR below 30 breaths/minute, which is high enough for normal adult patients, but much too low for smaller patients not even to mention neonates.

Furthermore the suctioning of the sample gas from the respiratory circuit and the patient's airway may cause a lung damage. This may happen due to a malfunction of one or more devices connected to a respiratory circuit or a user error. The lung may be damaged due to a blockage between the sidestream airway adapter used for the gas sampling and the ventilator, which causes insufficient flow of gas in to the patient, which in turn ends to suctioning of the sampling gas from the patient side of the respiratory circuit in other words from the patient's airways that may empty the lungs out of the gas causing lung collapse.

Thus, the existing sidestream gas analyzing is not suitable for small patients with high RR and small TV and in practice there does not exist a proper breathing gas concentration analyzing technique for smaller patients at the moment.

EP 2168623 discloses a method and arrangement for detecting a leak in anesthesia system. Both ventilator and fresh gas volume added for the inspiration and removed for an expiration are determined by a leak analyzer and the gas volume added and removed are compared to each other to determine the anesthesia system leakage.

US 5050615 discloses a method for determining the content of a gas component exhaled by the patient. The sample is withdrawn from the sampling location and moved along a tube incrementally towards an analyzer to determine the content of the gas component. These withdrawing and moving steps are repeated so that samples from each of plurality of successive respiratory cycles are collected one after the other inside the tube until they are analyzed.

### BRIEF DESCRIPTION OF THE INVENTION

The above-mentioned shortcomings, disadvantages and problems are addressed herein which will be understood by reading and understanding the following specification.

In an embodiment, an arrangement for maintaining a volume of a breathing gas in a desired level when ventilating a subject includes a ventilator for supplying along a first tubing a breathing gas for an inspiration and for receiving along a second tubing a breathing gas for an exhalation and a sample output connector for withdrawing a gas sample to a gas analyzer for an analysis from the breathing gas flowing along at least one of the first tubing and the second tubing. The arrangement for maintaining a volume of a breathing gas in a desired level when ventilating a subject also includes a measuring unit for providing a signal indicative of a volume of the gas sample withdrawn and a compensation gas inlet for adding a volume of a compensation gas into the breathing gas. The arrangement for maintaining a volume of a breathing gas in a desired level when ventilating a subject further includes a processing unit for determining the volume of the gas sample based on the signal provided by the measuring unit and for controlling the compensation gas inlet for adding the volume of the compensation gas into the breathing gas based on the volume of the gas sample withdrawn for maintaining the volume of the breathing gas in the desired level and to adjust sample gas volume proportional to the respiration rate or inversely proportional to the tidal volume provided by one of the gas analyzer and the ventilator.

Various other features, objects, and advantages of the invention will be made apparent to those skilled in art from the accompanying drawings and detailed description thereof.

### BRIEF DESCRIPTION OF THE DRAWINGS

Figure 1 is a schematic view of an arrangement in accordance with an embodiment; and

Figure 2 is a schematic view of an arrangement in accordance with another embodiment.

### DETAILED DESCRIPTION OF THE INVENTION

Specific embodiments are explained in the following detailed description making a reference to accompanying drawings. These detailed embodiments can naturally be modified and should not limit the scope of the invention as set forth in the claims.

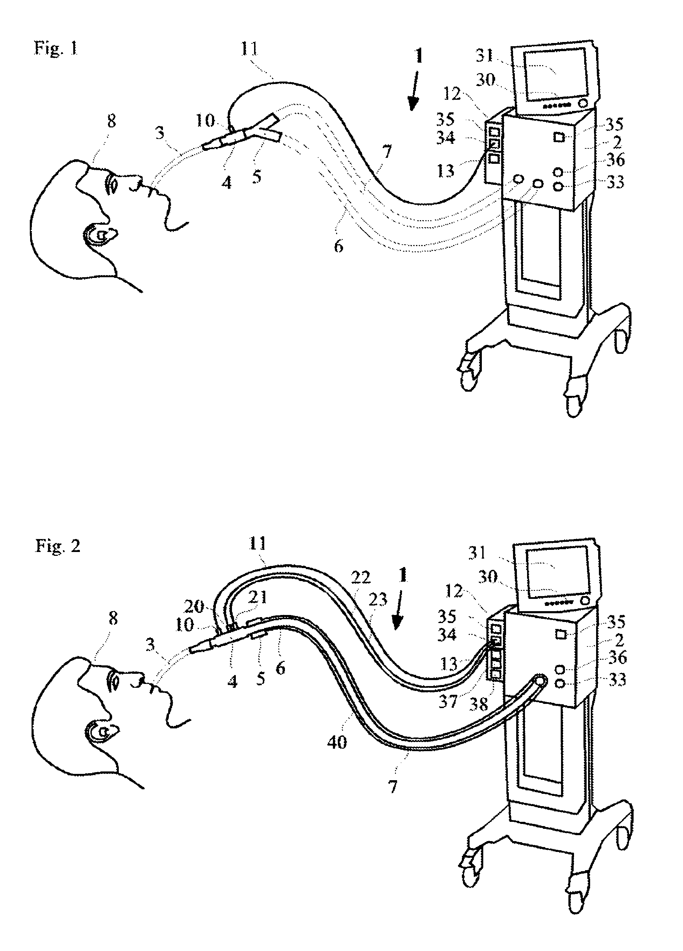

Fig. 1 shows a schematic view of an arrangement 1 for maintaining a volume of a breathing gas in a desired level when ventilating a subject 8. The arrangement comprises a ventilator 2 connected to the subject 8 for supplying along a first tubing 6 the volume of the breathing gas for an inspiration and for receiving along a second tubing 7 the volume of the breathing gas for an exhalation. Also the arrangement comprises a sample output connector 10 for withdrawing by means of a pump 13 a gas sample for an analysis from the breathing gas flowing along at least one of said first tubing 6 and the second tubing 7. The sample output connector 10 may be a part of an adapter 4 such as a conventional sidestream type airway adapter assembled in flow connection with the first tubing 6 and the second tubing 7 and the ventilator 2, typically between the ventilator 2 and an endotracheal tube 3 guiding both the breathing gas for the exhalation from lungs of the subject and the breathing gas for the inhalation to the lungs of the subject.

Also in Figure 1 there is between the adapter 4 and the ventilator 2 a branching unit 5 having three limbs, one of them being connected to the first tubing 6, another one being connected to the second tubing 7 and the third one being connected to the adapter 4. A sampling tube 11 is connected to the sample output connector 10 guiding a gas sample to a gas analyzer 12, such as a sidestream type gas analyzer, placed in Figure 1 outside the ventilator 2, but which could as well be inside the ventilator 2.

The arrangement further comprises a measuring unit 34 needed to measure the volume of the gas sample withdrawn through the sample output connector 10 and to provide a signal indicative of the volume of the gas sample withdrawn. The measuring unit 34 locates in the gas analyzer 12 in the embodiment of Figure 1, but could locate in any other suitable place, for instance it could be a part of the adapter 4. The measuring unit can be a flow sensor based on different flow measuring principles such as a hot wire anemometer, a differential pressure sensor, ultrasonic flow sensor etc.

The arrangement also comprises a compensation gas inlet 36 and a processing unit 35. The compensation gas inlet 36 adds a compensation gas into the breathing gas flowing between the ventilator 2 and the endotracheal tube 3 to compensate the volume of the gas sample withdrawn for the analysis. The compensation gas inlet 36, which may be a part of the ventilator 2, may comprise a valve or similar to allow a control of the compensation gas flow into the breathing gas.

The processing unit 35, which may be e.g. part of the gas analyzer 12 or the ventilator 2 as shown in Figure 1, determines the volume of the gas sample based on the signal provided by the measuring unit 34. Also the processing unit 35 is controlling the compensation gas inlet 36 for adding a volume of the compensation gas into the breathing gas based on the determined volume of the gas sample withdrawn for maintaining the volume of a breathing gas in a desired level. The volume of the compensation gas to be added into the breathing gas for maintaining the volume of the breathing gas in the desired level should be at least 50 %, more specifically at least 75 % or even more specifically substantially 100 % of the volume of the gas sample withdrawn. The processing unit 35 is able to compare the volume of the gas sample withdrawn with the desired level and use this information while controlling the volume of the compensation gas, which should be added into the breathing gas.

The arrangement may also comprise a user interface 30 allowing a user to set the desired level for the volume of the breathing gas, but the desired level may have been set already in the factory manufacturing the arrangement. A display 31 is useful when viewing various information received such as the volume and the concentration of various components of the gas sample and parameters indicating the condition of the subject 8.

A schematic view of another arrangement is shown in Figure 2. The subject 8 is connected to the ventilator 2 via a conventional coaxial tubing 40 comprising both the first tubing 6 for the inspiration, which is an inner one, and the second tubing 7 for the expiration, which is an outer one. The first and second tubings are operationally connected to the endotracheal tube 3. The inspiratory and expiratory breathing gas flows through the branching unit 5 just as explained with Figure 1 in the end of the coaxial tubing 40. The design of this branching unit is slightly different from the design of Figure 1, but its basic function is same. Also this arrangement shown in Figure 2 comprises the adapter 4 with the sample output connector 10 for the sampling tube 11 for withdrawing the gas sample to the gas analyzer 12 placed inside or outside the ventilator 2. In addition there is at least a first port 20 for measuring a pressure prevailing between the endotracheal tube 3 and the ventilator 2. In case the pressure is measured by the pressure sensor 37 at a distance from the first port 20 as shown in Figure 2, there is needed a third tubing 22 to conduct the pressure for the measurement. Advantageously the adapter 4 is equipped with the first pressure port 20. To measure a flow between the endotracheal tube 3 and the ventilator 2 exploiting the pressure difference technique also a second port 21 is needed for the flow communication. The second port 21, which may also be in the adapter 4, is connected to a fourth tubing 23. The pressure difference is measured between the first ports 20 and the second port 21 over the flow barrier (not shown in figures) by the flow sensor 38. Naturally the flow of the breathing gas can be measured without pressure ports with other measuring principles, such as a hot wire technique, too. Signals provided by the pressure sensor 37 and the flow sensor 38 are received by the processing unit 35 to determine the pressure and the flow rate of the breathing gas, which information can be exploited by the user or the processing unit 35 when withdrawing the gas sample from the breathing gas.

To imitate the normal breathing of the subject during the inspiration the ventilator 2 pushes the fresh inspiratory breathing gas through the first tubing 6, the branching unit 5, the adapter 4 and the endotracheal tube 3 into the subject's respiratory system. Similarly after the compliance of subject's lungs during the expiration the ventilator 2 allows to release the pressure inside the first tubing 6, the branching unit 5, the adapter 4, the endotracheal tube 3 and the subject's respiratory system by opening an expiration valve 33. The gas sample withdrawn along the sampling tube 11, which gas sample may include the breathing gas for the inspiration and expiration flowing between the subject and the ventilator 2, is typically used to analyze its concentration. Withdrawing may be carried out by sucking the gas sample through the sample output connector 10 and the sampling tube 11 into the gas analyzer 12. The volume of the gas sample withdrawn from the breathing gas flow depends on the flow rate of the gas sample through the output connector 10 of the adapter 4. The higher the RR and the smaller the tidal volume (TV) of the subject, the higher the flow rate or the volume of the gas sample is needed to minimize averaging of gas samples and to enable as accurate as possible gas concentration measurement.

The gas sample flow out from the volume of the breathing gas flowing between the subject and the ventilator 2 decreases the volume of the breathing gas entering the subject's lungs within the inspiration. To ensure that the subject gets enough the breathing gas in to the lungs and that the subject is ventilated sufficiently, the volume of breathing gas during the inspiration period, that is lost in to the gas sampling, needs to be compensated by adding through the compensation gas inlet 36 a sufficient volume of the compensation gas, such as fresh gas, which may include air, oxygen and if necessary some narcotic agent, into the breathing gas, which compensation gas may be delivered through the hospital gas delivery system. The compensation gas can be also a mixture of some of these gases. For example in a closed loop ventilation system the compensation gas usually includes much higher concentration of oxygen, but also other gases, compared to the open loop ventilation system where the compensation gas may include just filtered room air. The sufficient volume of the compensation gas is needed to maintain the volume of the breathing gas in the desired level. The compensation gas can be added during the inspiratory phase of the ventilation to compensate the gas sample withdrawn during the inspiratory phase, too.

The measuring unit 34 shown in Figure 1 and 2 is able to measure the flow rate of the gas sample flowing through the sampling tube 11 and the gas analyzer 12, which is based on the sidestream technique. Furthermore the respiration rate (RR) can be measured from the alternating gas concentration or capnogram produced by the gas analyzer 12. When a normal adult, whose tidal volume (TV) is 500 ml, RR 15 and the inspiration volume:expiration volume (I:E) ratio between the inspiration and the expiration 1:2, a sample gas flow of 200 ml/min is sufficient, with a sample tube which length is 3 meters and inner diameter 1.2 mm, to achieve a reasonable sensitivity and accuracy for the gas concentration measurement. However, to achieve even some sensitivity for the gas concentration measurement at higher RR and smaller TV, for example 2 kg neonate whose RR is about 100 breaths/min, TV about 10 ml and I:E ratio 1:1, a sample gas flow of 400 ml/min is needed, with a sample tube which length is 3 meters and inner diameter 1.2 mm.

The flow rate of the gas sample or in other words the volume of gas drawn from the breathing gas by the sample gas flow strongly depends on RR and I:E ratio, which information is provided to the processing unit 35. The volume of the gas drawn from the breathing gas during one inspiration can be calculated by multiplying the flow rate of the gas sample by the time of inspiration. The flow rate of the gas sample also depends on the inner diameter of the sampling tube 11, so that decrement in tube diameter increases the sample flow rate. However, much more work needs to be done to suck the gas sample through the sampling tube 11 with a smaller diameter and the situation gets even worse as the tube is clocked up by a water, mucus and other secretions, which is common during care especially with tubes having a smaller inner diameter. Thus it is an established practice to use sample tubes with an inner diameter between 1-1.5 mm or preferably 1-1.2 mm to minimize clocking up of the sample tubes, but to achieve as fast gas sample flow rates as possible. The information of I:E ratio of the subject can be delivered from the ventilator 2 or it can be measured with an arrangement used to measure the pressure and the flow of the breathing gas in the breathing circuit as shown and explained in Figure 2.

On the other hand RR is inversely proportional to TV and the size of the subject. The volume of the gas that is witdrawn by the sidestream gas sampling during the inspiration can be compensated by increasing the inspiratory compensation gas volume that the ventilator pushes in to the patient. The ventilator 2 controls the RR and I:E ratio of a ventilated subject, but also receives the information of the gas sample flow rate from the measuring unit 34, which information is needed for the compensation. The volume of the gas sampled during the inspiration for the adult, whose RR is 15 breaths/minute and I:E ratio 1:2 is about 4.4 ml, based on which volume of the compensation gas the ventilator should add in to the inspired breathing gas to ensure sufficient ventilation. Similarly the volume of the gas sample during the inspiration for 2 kg neonate whose RR is about 100 breaths/min and I:E ratio 1:1 is proportionally about 2 ml, which is about 1/5 of the subject's TV. Again, the ventilator should add the volume of the compensation gas based on this volume of the gas sample in to the inspired gas to ensure sufficient ventilation of that small subject.

Existing ventilators may be very accurate and very controllable. The measuring unit 34 in the gas analyzer can measure the flow rate or volume of the gas sample withdrawn from the subject's inspiratory breathing gas flow, which is also the flow rate of the gas sample through the gas analyzer 12. The flow rate information is used to control the pump withdrawing the gas sample through the sample output connector 10 to maintain a constant sample flow through the gas analyzer 12 to get accurate gas concentration values. Thus the ventilator 2 can compensate the loss of the breathing gas for the inspiration taken by the gas analyzer 12 in the form of the gas sample by adding the same volume or sufficient volume as described hereinbefore of the compensation gas during the inspiration. This is achieved by providing the information about the volume of the gas sample withdrawn for the analysis of the gas analyzer 12 to the ventilator 2, which uses it to compensate the volume of the gas sample withdrawn within the inspiration by adjusting ventilation parameters. The benefit is that higher gas sample flows can be used to enable an accurate gas concentration measurement at higher respiration rates without interfering the gas exchange and the ventilation of the lungs.

It is also possible to implement an adjustable gas sample flow for example between 50 and 500 ml/min to enable the measurement of high RR of small patients. Since it is beneficial to use higher gas sample flows (>200 ml/min) to ensure an accurate gas concentration measurement, it is desirable to adjust the gas sample flow or volume proportional to the respiration rate or inversely proportional to the tidal volume automatically. Thus as the respiration rate increases, the gas sample flow or volume is also increased or as the tidal volume decreases, the gas sample flow or volume is increased. Similarly the information of increase/decrease of gas sample flow or volume can be used by the ventilator 2 controlling RR to automatically compensate the volumetric change of the gas sample taken by the sidestream gas analyzer to increase/decrease the volume of the compensation gas added into the breathing gas flow for the inspiration.

The adjustable flow of the gas sample can also be used when measuring non-intubated patients to increase the gas concentration measurement accuracy at higher RR. This can be implemented by using the flow rate of the gas sample, but also the RR information that the processing unit 35 calculates itself. Thus as RR increases, the flow rate of the gas sample is increased at the same time to prevent averaging of the gas samples and to enable the fast and accurate gas concentration measurement.

The volume of the compensation gas that the ventilator adds may be calculated from the information of the respiration rate (RR) from the ventilator 2 and the flow rate of the gas sample from the measuring unit 34. Thus the total volume of the breathing gas that flows into the subject's lungs remains in the desired level regardless of the volume of the gas sample withdrawn. This is achieved by sending the flow rate information of the gas sample from the processing unit 35 of the gas analyzer 12 to the processing unit of the ventilator 2 unless they have a common processing unit, which can then adjust the volume of the breathing gas for the inspiration for compensation based on RR and the gas sample flow rate.

It is a current care practice in hospitals that the subject's breathing circuit is opened, by disconnecting the branching unit 5 or the adapter 4 of the arrangement 1 from the endotracheal tube 3, many times during the day because of different care procedures, such as suctioning of secretions and water from the subject's lungs or delivery of drugs in to the lungs with a nebulizer (not shown in figures) or to view the airways of the subject with fiber optics (not shown in figures). In addition to a harm and risks that relate to given care procedures it is always possible that there is an occlusion or blockage between the subject and the ventilator 2, typically in the first tubing 6 or the second tubing 7 between the sample output connector 10 of the adapter 4 and the ventilator 2. The reason may be a malfunction of one or more devices or a user error. In such case to prevent additional harm caused by the negative pressure generated by the gas sample flow of the gas analyzer in the subject's airways that would squeeze the lungs empty of the breathing gas the embodiment shown in Figure 2 can be used to prevent the alveoli to collapse deep in the lung that enable the gas exchange of the subject.

To ensure that the gas sample flow for the analysis does not generate a negative pressure into the subject's lungs, a signal is provided indicating at least one of the breathing gas flow measured by the flow sensor 38 and the pressure measured by the pressure sensor 37 through the first port 20 and the second port 21 and determined by the processing unit 35 to control the flow or the volume of the gas sample. In the case there is the occlusion or blockage between the endotracheal tube 3 and the ventilator 2, typically between the adapter 4 and ventilator 2 or between the ventilator 2 and one of the sample output connector 10, the first port 20 and the second port 21, the flow measured through the first port 20 and the second port 21 of adapter 4 approaches zero or close to zero as well as the pressure measured through the first port 20 approaches zero. Based on at least one of the flow and pressure information, withdrawing the gas sample is decreased or stopped to prevent negative pressures inside the lungs. The information can also be sent to the ventilator 2 to open the expiration valve 33 and of course to alarm the hospital personnel.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. An arrangement for maintaining a volume of a breathing gas in a desired level when ventilating a subject comprising:
a ventilator (2) for supplying along a first tubing (6) a breathing gas for an inspiration and for receiving along a second tubing (7) a breathing gas for an exhalation;
a sample output connector (10) for withdrawing a gas sample to a gas analyzer (12) for an analysis from the breathing gas flowing along at least one of said first tubing and said second tubing;
a measuring unit (34) for providing a signal indicative of a volume of the gas sample withdrawn;
a compensation gas inlet (36) for adding a volume of a compensation gas into the breathing gas, and
a processing unit (35) for determining the volume of the gas sample based on the signal provided by said measuring unit, and for controlling said compensation gas inlet for adding the volume of the compensation gas into the breathing gas based on the volume of the gas sample withdrawn for maintaining the volume of the breathing gas in the desired level, **characterized in that** said processing unit is configured to adjust sample gas volume proportional to the respiration rate or inversely proportional to the tidal volume.

2. The arrangement according to claim 1, further comprising a user interface (30) for setting said desired level for said volume of the breathing gas.

3. The arrangement according to claim 1, further comprising a measuring unit (34) for detecting a volume of the gas sample withdrawn.

4. The arrangement according to claim 1, **characterized in that** said processing unit (35) compares the volume of the gas sample withdrawn with the desired level of the breathing gas.

5. The arrangement according to claim 1, **characterized in that** said processing unit (35) controlling said compensation gas inlet (36) adds the volume of the compensation gas which is at least 50 %, more specifically at least 75 % or even more specifically substantially 100 % of the volume of said gas sample withdrawn.

6. The arrangement according to claim 1, **characterized in that** said processing unit (35) controlling said compensation gas inlet (36) adds the volume of the compensation gas into the breathing gas for the inspiration.

7. The arrangement according to claim 1, **characterized in that** said processing unit (35) determines the volume of the gas sample withdrawn during supplying the breathing gas for the inspiration.

## Patentansprüche

1. Anordnung zum Halten eines Volumens eines Atemgases auf einem gewünschten Niveau bei Beatmung einer Person, umfassend:
ein Beatmungsgerät (2) zum Zuführen, entlang einer ersten Schlauchleitung (6), eines Atemgases wegen einer Einatmung und zum Aufnehmen, entlang einer zweiten Schlauchleitung (7), eines Atemgases wegen einer Ausatmung;
einen Probenausgabeanschluss (10) zum Entnehmen einer Gasprobe, für einen Gasanalysator (12) zwecks einer Analyse, aus dem Atemgas, das entlang zumindest einer von der ersten Schlauchleitung und der zweiten Schlauchleitung strömt;
eine Messeinheit (34) zum Bereitstellen eines Signals, das ein Indikator für ein Volumen der entnommenen Gasprobe ist;
einen Ausgleichsgaseinlass (36) zum Hinzugeben eines Volumens eines Ausgleichsgases in das Atemgas, und
eine Verarbeitungseinheit (35) zum Bestimmen des Volumens der Gasprobe auf Grundlage des von der Messeinheit bereitgestellten Signals, und zum Steuern bzw. Regeln des Ausgleichsgaseinlasses zwecks Hinzugebens des Volumens des Ausgleichsgases in das Atemgas auf Grundlage des Volumens der entnommenen Gasprobe, um das Volumen des Atemgases auf dem gewünschten Niveau zu halten, **dadurch gekennzeichnet, dass** die Verarbeitungseinheit konfiguriert ist, um das Probengasvolumen proportional zur Atmungsrate oder umgekehrt proportional zum Atemzugvolumen zu regulieren.

2. Anordnung nach Anspruch 1, die weiterhin eine Benutzerschnittstelle (30) zum Einstellen des gewünschten Niveaus für das Volumen des Atemgases umfasst.

3. Anordnung nach Anspruch 1, die weiterhin eine Messeinheit (34) zum Erfassen eines Volumens der entnommenen Gasprobe umfasst.

4. Anordnung nach Anspruch 1, **dadurch gekennzeichnet, dass** die Verarbeitungseinheit (35) das Volumen der entnommenen Gasprobe mit dem gewünschten Niveau des Atemgases vergleicht.

5. Anordnung nach Anspruch 1, **dadurch gekennzeichnet, dass** die den Ausgleichsgaseinlass (36) steuernde bzw. regelnde Verarbeitungseinheit (35) das Volumen des Ausgleichsgases hinzugibt, das zumindest 50%, spezieller zumindest 75% oder noch spezieller im Wesentlichen 100% des Volumens der entnommenen Gasprobe beträgt.

6. Anordnung nach Anspruch 1, **dadurch gekennzeichnet, dass** die den Ausgleichsgaseinlass (36) steuernde bzw. regelnde Verarbeitungseinheit (35) das Volumen des Ausgleichsgases in das Atemgas für die Einatmung hinzugibt.

7. Anordnung nach Anspruch 1, **dadurch gekennzeichnet, dass** die Verarbeitungseinheit (35) das Volumen der Gasprobe bestimmt, die während des Zuführens des Atemgases für die Einatmung entnommen wird.

## Revendications

1. Agencement destiné à maintenir un volume de gaz de respiration à un niveau désiré au cours de la ventilation d'un sujet comprenant :
un ventilateur (2) destiné à délivrer, sur une première tuyauterie (6), un gaz de respiration pour une inspiration et à recevoir sur une seconde tuyauterie (7) un gaz de respiration pour une exhalation ;
un raccord de sortie d'échantillon (10) destiné à extraire un échantillon de gaz vers un analyseur de gaz (12) afin d'assurer une analyse du gaz de respiration circulant sur au moins l'une de ladite première tuyauterie et ladite seconde tuyauterie ;
une unité de mesure (34) destinée à produire un signal représentatif d'un volume de l'échantillon de gaz extrait ;
une entrée de gaz de compensation (36) destinée à ajouter un volume d'un gaz de compensation dans le gaz de respiration, et
une unité de traitement (35) destinée à déterminer le volume de l'échantillon de gaz sur la base du signal délivré par ladite unité de mesure, et à commander ladite entrée de gaz de compensation afin d'ajouter le volume du gaz de compensation dans le gaz de respiration sur la base du volume de l'échantillon de gaz extrait afin de maintenir le volume du gaz de respiration au niveau désiré, **caractérisé en ce que** ladite unité de traitement est configurée afin d'ajuster le volume de gaz échantillon de manière proportionnelle au débit de respiration ou inversement proportionnelle au volume périodique.

2. Agencement selon la revendication 1, comprenant, en outre, une interface d'utilisateur (30) afin de positionner ledit niveau désiré dudit volume de gaz de respiration.

3. Agencement selon la revendication 1, comprenant, en outre, une unité de mesure (34) afin de détecter un volume de l'échantillon de gaz extrait.

4. Agencement selon la revendication 1, **caractérisé en ce que** ladite unité de traitement (35) compare le volume de l'échantillon de gaz extrait avec le niveau désiré du gaz de respiration.

5. Agencement selon la revendication 1, **caractérisé en ce que** ladite unité de traitement (35) commandant ladite entrée de gaz de compensation (36) ajoute le volume du gaz de compensation qui représente au moins 50 %, de manière plus spécifique, au moins 75 % ou de manière encore plus spécifique, sensiblement 100 % du volume dudit échantillon de gaz extrait.

6. Agencement selon la revendication 1, caractérisé en ce ladite unité de traitement (35) commandant ladite entrée de gaz de compensation (36) ajoute le volume du gaz de compensation dans le gaz de respiration pour l'inspiration.

7. Agencement selon la revendication 1, **caractérisé en ce que** ladite unité de traitement (35) détermine le volume de l'échantillon de gaz extrait au cours de la fourniture du gaz de respiration pour l'inspiration.
